# FASCICULE DE BREVET EUROPEEN

(11) **EP 3 349 858 B1**
(45) Date de publication et mention de la délivrance du brevet: **08.01.2020**
(21) Numéro de dépôt: 16777713.5
(22) Date de dépôt: 05.09.2016
(51) Int. Cl.: A61Q 3/02, A61K 8/73, A61K 8/891

(54) **COMPOSITION DE VERNIS À ONGLES À BRILLANCE AMÉLIORÉE**
HOCHGLANZ NAGELLACK
NAIL POLISH WITH IMPROVED GLOSS

(30) Priorité: 15.09.2015 FR 1558624
(43) Date de publication de la demande: 25.07.2018
(73) Titulaire: Fiabila, 28130 Maintenon (FR)
(72) Inventeur: NOUGUEREDE, Olivier, 28130 Hanches (FR)
(74) Mandataire: Le Cloirec, Claudine
(86) Numéro de dépôt international: PCT/FR2016/052194
(87) Numéro de publication internationale: WO 2017/046473

(56) Documents cités:
- WO-A1-2014/176275
- US-A- 5 302 379
- US-A- 5 374 674

## Description

La présente invention concerne le domaine des vernis à ongles, et plus particulièrement les compositions de vernis à ongles à brillance améliorée.

Les compositions de vernis à ongles renferment de manière classique un solvant, qui peut être un solvant organique ou aqueux, un plastifiant et au moins un agent filmogène. Pour les formules colorées contenant des pigments et/ ou des nacres un ou des agents thixotropants sont ajoutés. Ces vernis, de par leur faible viscosité, sont appliqués en une ou plusieurs couches minces sur les ongles. Le séchage par évaporation du solvant conduit en quelques minutes à la création d'un film qui adhère à l'ongle et durcit.

Les principales propriétés recherchées par les utilisateurs pour ces vernis appliqués sur l'ongle sont leur adhérence et leur tenue sur l'ongle, leur dureté, leur flexibilité mais également, pour ces produits cosmétiques, leur brillance. Les fabricants doivent donc proposer des compositions de vernis permettant un bon compromis entre toutes ces propriétés.
Par ailleurs, il est également nécessaire que ces compositions de vernis, qui sont généralement vendues en flacons transparents pour permettre d'en vérifier la teinte, soient le moins possible sujettes au jaunissement et ne présentent aucun phénomène de séparation de phase ou de démixtion des couleurs (correspondant à un phénomène de synérèse) lors de leur stockage, pouvant suggérer une mauvaise qualité du vernis.

Pour améliorer la brillance des vernis, il a été proposé dans le document FR 2 823 108 A1 de la société l'Oréal un dérivé de sulfonamide en tant que plastifiant dans une formulation de vernis présentant également une résistance à l'usure améliorée. Cependant ce document ne mentionne aucune valeur de brillance. De plus cette composition de vernis ne possède pas de stabilité au stockage en raison de l'apparition de synérèse dans le flacon.

On connaît également du document EP 1 900 352 A1 l'introduction de résines cétone/aldéhyde pour augmenter la brillance de compositions de vernis à ongles renfermant de la nitrocellulose et des agents épaississants, tels que des bentones destinées à améliorer la stabilité dans le temps de ces compositions, mais qui présentent l'inconvénient d'engendrer une matification du film de vernis à ongles. Cependant, comme cela sera montré plus loin dans les exemples comparatifs, un fort jaunissement de ces compositions renfermant des cétone/aldéhyde est observé, ce qui est rédhibitoire lorsqu'elles sont conditionnées dans des flacons transparents.

De même, la société Revlon a décrit dans la demande de brevet internationale WO 97/42930 l'utilisation d'une résine acrylique comprenant de l'acéto acétoxy éthyl méthacrylate dans une composition de vernis à ongles renfermant une faible proportion de nitrocellulose. Ce document indique que la brillance du vernis est améliorée, mais sans donner de valeurs de brillance. De plus, de telles compositions comportant des composés de type (méth)acrylate présentent une forte tendance au jaunissement, ce qui est rédhibitoire pour des vernis à ongles.

On connaît de FR 2.916.965 des compositions de vernis à ongles renfermant une faible teneur en nitrocellulose (inférieure à 5 % ou inférieure à 1 %) et une résine siliconée phénylée, principalement une résine alkyl phényl silsequioxane. On connaît également de FR 2.939.661 des compositions de vernis à ongles renfermant une teneur en nitrocellulose inférieure à 1 %, voire sans nitrocellulose, une résine siliconée phénylée et un polyuréthane.

De telles compositions renfermant de faibles teneurs en agent filmogène nitrocellulose présentent notamment un déficit d'adhésion sur l'ongle, ainsi qu'une faible tenue de la brillance dans le temps.

Par ailleurs, la demande WO 2014/176275 divulgue une composition de vernis pour les ongles qui, outre des solvants et au moins 6 % d'agent filmogène à base de nitrocellulose, peut renfermer une résine polyphénylsilsesquioxane (AM0281). La demande de brevet américain US 2004/0180011 A1 décrit l'utilisation de résine alkyl phényl silsesquioxane pour des produits cosmétiques autres que des vernis pour les ongles, notamment des formulations ne renfermant pas d'agent filmogène et/ou ne renfermant pas de plastifiant, tels que des mascaras, des rouges à lèvres, des lotions pour le corps, ou encore des produits de soin pour les cheveux. En effet, certains agents filmogènes, tels que les dérivés de cellulose, et plus particulièrement la nitrocellulose, ont tendance à se déstabiliser en présence d'autres composés. En aucun cas les questions du jaunissement de la formulation ou d'observation de synèrèse ne peuvent se poser pour de telles compositions, qui ne sont notamment pas conditionnées dans des flacons transparents.

Un but de la présente invention est donc de pallier les inconvénients des vernis à ongles de l'art antérieur, en proposant une composition de vernis à ongles, destinée à être présentée aux utilisateurs dans des flacons transparents, offrant une brillance améliorée du vernis après son application sur les ongles, et une tenue dans le temps de cette brillance sur les ongles.

Un autre but de l'invention est de proposer une composition de vernis à ongles, notamment une composition de vernis à ongles renfermant un agent filmogène à base de nitrocellulose, présentant une brillance améliorée du vernis, sans provoquer de jaunissement lors de son stockage dans un flacon transparent, ni de phénomène de synérèse au repos, même après plusieurs mois de stockage.

A cet effet la présente invention concerne une composition de vernis pour les ongles, renfermant au moins un agent filmogène, à base de nitrocellulose et/ou d'un dérivé de cellulose, un plastifiant et un solvant, caractérisée en ce qu'elle comprend au moins 6 % massique d'agent filmogène à base de nitrocellulose et/ou d'un dérivé de cellulose et une résine polyphénylsilsesquioxane, en vue d'améliorer la brillance du vernis après son application sur les ongles, ladite résine polyphénylsilsesquioxane étant une molécule non aminée et présentant une température de transition vitreuse Tg supérieure à 30°C.

Avec une telle résine, il a été constaté de manière surprenante, que la brillance du vernis appliqué sur les ongles est augmentée, sans provoquer de jaunissement, ni déstabiliser la formulation renfermant au moins 6 % massique de nitrocellulose ou de dérivé de cellulose. La composition de vernis peut avantageusement renfermer une teneur en nitrocellulose ou dérivé de cellulose supérieure ou égale à 8 % massique, voire supérieure ou égale à 10 % massique.

De préférence, la masse molaire de la résine polyphénylsilsesquioxane est comprise entre 500 et 20 000 g/mol, de préférence comprise entre 1 000 et 10 000 g/mol, de préférence encore comprise entre 1 500 et 5 000 g /mol.

Le solvant de la composition de vernis à ongles comprend avantageusement un solvant organique, dans lequel est soluble la résine polyphényl silsesquioxane, ledit solvant organique étant de préférence choisi parmi les acétates, tels que les acétates d'alkyle.

De préférence, le solvant organique, dans lequel est soluble la résine polyphényl silsesquioxane, comprend l'acétate d'éthyle et /ou l'acétate de butyle.

Ladite résine poly phényl silsesquioxane, présentant une Tg supérieure à 30°C, est un solide à 25°C. Elle présente avantageusement une température de transition vitreuse Tg supérieure à 35 °C, de préférence supérieure à 40°C. Une telle température de transition vitreuse permet de conserver des propriétés de dureté suffisante pour ne pas impacter la tenue du film de vernis sur l'ongle.

Par rapport à l'art antérieur qui met en oeuvre des résines renfermant des unités alkyl phenyl silsesquioxane, la résine polyphénylsilsesquioxane selon l'invention est formée de plus de 98 % d'unités phénylsilsesquioxane par rapport au nombre total d'unités silsesquioxane de la résine.

De manière préférée, la composition de vernis selon l'invention renferme une proportion massique en résine polyphényl silsesquioxane comprise entre 0,1 % et 10 %, de préférence comprise entre 0,5 % et 5 %, de préférence encore entre 1 % et 5 % de la masse totale de la composition de vernis.

La composition de vernis comprend avantageusement un premier agent filmogène (également dénommé agent filmogène primaire) choisi parmi la nitrocellulose ou un dérivé de la cellulose, et un deuxième agent filmogène (également dénommé agent filmogène secondaire, en raison de sa plus faible concentration, dans la composition de vernis, que la concentration de l'agent filmogène primaire) choisi parmi les résines polyester et/ou epoxy/tosylamide et/ou (méth)acrylique. Parmi ces agents filmogènes, on évitera en particulier le polyuréthane, notamment à des concentrations supérieures à 0,5 % massique. Il a, en effet, été constaté que l'association polyuréthane et nitrocellulose ou dérivé de cellulose provoque un jaunissement important et une dégradation de l'adhésion du vernis sur l'ongle.

Selon un mode de réalisation préféré de l'invention, la composition de vernis renferme au moins un solvant choisi parmi les acétates d'alkyle, un premier agent filmogène choisi parmi la nitrocellulose ou un dérivé de la cellulose, un deuxième agent filmogène polyester comprenant par exemple le copolymère anhydride triméllitique/acide adipique/néopentyl glycol, et une proportion massique en polyphényl silsesquioxane comprise entre 0,1 % et moins de 10 % de la masse totale de la composition de vernis.

Plus particulièrement, la composition de vernis peut comprendre de de 60 à 90 % massique de solvant, de 6 à 30 % massique du premier agent filmogène, de 2 à 20 % massique du deuxième agent filmogène et de 0,1 % à moins de 10 % massique, de préférence de 0,5 à 5 % massique, de polyphényl silsesquioxane, par rapport à la masse totale de la composition de vernis.

Selon mode de réalisation préféré de l'invention la composition de vernis comprend de 60 à 80 % massique de solvant, de 8 à 25 % massique du premier agent filmogène, de 5 à 15 % massique du deuxième agent filmogène et de 0,1 % à 9 % massique, de préférence de 0,5 à 5 % massique, de polyphényl silsesquioxane, par rapport à la masse totale de la composition de vernis.

Avantageusement, la composition de vernis, selon la présente invention, renferme une teneur en benzène inférieure à 2 ppm, de préférence inférieure à 1 ppm.

La présente invention va maintenant être décrite plus en détail et illustrée par les exemples, non limitatifs, ci-après.

### EXEMPLES

### Préparation de la composition :

Tous les essais présentés ci-après (à l'exception du test de stabilité à la synérèse) mettent en oeuvre une composition de vernis à ongles de formulation indiquée dans le tableau 1 ci-dessous (tous les pourcentages sont des pourcentages massiques de la composition totale).

**Tableau 1**

| | |
|---|---|
| Acétate de butyle | 32,5 - x/2 |
| Acétate d'éthyle | 33 - x/2 |
| Nitrocellulose | 15 |
| Acétyl tributyl citrate | 6 |
| Copolymère acide adipique/néopentyl glycol/anhydride triméllitique | 12 |
| Résine | x |
| Bentonite de stearalkonium | 1,5 |
| **Total** | **100** |

### Test réalisés :

- Brillance : sur une plaque de type Leneta, on applique une couche de 100 µm d'épaisseur de la composition de vernis. Après séchage à 20 °C du film obtenu, on mesure sa brillance (Gloss, UB) sous un angle d'incidence de 60° à l'aide d'un brillancemètre Byk Gardner.
- Flexibilité : sur une plaque en aluminium recouverte d'un vernis humide de 300 µm d'épaisseur, on réalise un emboutissage lent et on mesure la profondeur de la pénétration (selon la norme ISO1520). Une valeur supérieure à 3,0 est souhaitée, préférentiellement supérieure à 4,0.
- Dureté : la dureté Persoz est mesurée sur une plaque de verre recouverte d'un vernis de 100 µm humide (selon la norme ISO1522). Une valeur minimale de 200 est souhaitée, préférentiellement supérieure à 210.
- Adhérence : le Cross hatch test est réalisé sur une plaque de verre. Un film de 100µm humide est appliqué et séché 24h à 20°C. Un quadrillage est réalisé au moyen d'un couteau à plusieurs lames. Un ruban adhésif est collé sur ce quadrillage, puis retiré : on analyse alors le quadrillage. La note 0 correspond à l'absence de perte d'adhésion (aucun carré n'a été enlevé). La note 5 correspond à la perte totale d'adhésion (tous les carrés ont été enlevés). Une note comprise entre 0 et 1 est indispensable.
- Stabilité :
   ∘ Stabilité au jaunissement : le vernis incolore est placé dans une étude à 45 °C durant 1 mois. Il est retiré et comparé visuellement à un produit placé à une température ambiante. Le jaunissement est plus particulièrement étudié.
   ∘ Stabilité à la synérèse : un vernis teinté (renfermant des pigments) est placé à l'étuve à 45 °C durant 30 jours. Les phénomènes d'exsudation sont observés. Aucun phénomène de séparation de phase ni de démixtion de couleur ne doit être observé. Le phénomène d'exsudation doit être le plus faible possible.

Ce dernier test de stabilité à la synérèse est effectué avec une composition de vernis à ongles teinté renfermant du dioxyde de titane et un pigment de couleur selon la formulation indiquée dans le tableau 2 ci-après.

**Tableau 2**

| | |
|---|---|
| Acétate de butyle | 32,3 - x/2 |
| Acétate d'éthyle | 32 - x/2 |
| Nitrocellulose | 15 |
| Acétyl tributyl citrate | 6 |
| Copolymère acide adipique/néopentyl glycol/anhydride triméllitique | 12 |
| Résine | x |
| Bentonite de stearalkonium | 1,5 |
| Dioxyde de titane | 1 |
| Pigment Red 7 lake | 0,2 |
| **Total** | **100** |

### Exemple 1 :

Différents taux x de résine poly phényl silsesquioxane (résine DC 217 de la société Dow Corning) ont été testés dans les essais 1 à 6 présentés dans le tableau 3 ci-après.

Cette résine est un polymère siloxane à base essentiellement d'unités phényl silsesquioxane (c'est-à-dire renfermant moins de 2 % d'autres unités siloxane en tant de constituants indésirables) se présentant, à 25°C, sous la forme d'un solide transparent.

Cette résine poly phényl silsesquioxane possède les propriétés suivantes :

| | |
|---|---|
| Tg : | 64 °C |
| Extrait sec : | 100 % |
| Viscosité à 150°C : | 1 400 cP |
| Masse Moléculaire : | 1500-2200 g / mol |

Elle est soluble dans le mélange de solvants acétate d'éthyle et acétate de butyle.

**Tableau 3**

| | **Essai 1** | **Essai 2** | **Essai 3** | **Essai 4** | **Essai 5** | **Essai 6** |
|---|---|---|---|---|---|---|
| **X (% massique)** | 0 | 0,5 | 1 | 2,5 | 5 | 10 |
| **Gloss (60°, UB)** | 87,4 | 89 | 89,5 | 90 | 91,2 | 91 |
| **Stabilité au jaunissement** | Faible jaunissement | Faible jaunissement | Faible jaunissement | Faible jaunissement | Faible jaunissement | Faible jaunissement |
| **Stabilité à la synérèse** | Stable | Stable | Stable | Stable | Stable | Stable |
| **Dureté (s)** | 219 | 214 | 216 | 228 | 224 | 178 |
| **Flexibilité (mm)** | 5,7 | 5,6 | 5,3 | 5,3 | 5,4 | 6 |
| **Adhérence** | 0 | 0 | 0 | 0 | 0 | 0 |

Toutes ces compositions renferment moins de 1 ppm de benzène.

Par rapport à la composition de référence (notée essai 1), sans résine de poly phényl silsesquioxane, les vernis renfermant cette résine (essais 2 à 6) présentent une brillance améliorée sous un angle d'incidence de 60°.

Leur jaunissement est faible après un mois à 45°C, et aucune synérèse n'est observée dans le flacon. La composition de vernis présente donc, dans son flacon, un aspect toujours attrayant pour l'utilisateur.

De manière surprenante, l'ajout de cette résine poly phényl silsesquioxane ne perturbe pas l'équilibre de la composition de vernis et n'affecte pas non plus les propriétés du vernis qui reste flexible, dur et adhère correctement à l'ongle.
Néanmoins une teneur de 10 % massique affecte la dureté du vernis qui est un peu faible.

Un test a été réalisé sur un panel de 20 personnes. Un premier groupe a testé le vernis Essai 1, un second groupe le vernis Essai 3 et un troisième groupe le vernis Essai 4. Ces panelistes ont donné leur avis sur la tenue de la brillance du vernis appliqué. La moyenne des réponses est présentée dans le tableau 4 ci-dessous.

**Tableau 4**

| **Vernis** | **Tenue de la brillance** |
|---|---|
| Vernis Essai 1 | 4,6 jours |
| Vernis Essai 3 | 4,5 jours |
| Vernis Essai 4 | 4,6 jours |

Ces résultats indiquent que la tenue de la brillance n'est pas détériorée par l'ajout de la résine polyphénylsilsesquioxane dans la composition de vernis.

### Exemple 2 (comparatif) : Essais comparatifs par rapport à l'état de l'art

En remplacement de la résine x présentée dans les tableaux 1 et 2, ont été testés la résine cétone/aldéhyde du brevet EP 1.900.352 et le dérivé de sulfonamide du brevet FR 2.823.108. les résultats sont présentés dans le tableau 5.

**Tableau 5**

| | **Essai 1** | **Essai 7 (comparatif)** | **Essai 8 (comparatif)** |
|---|---|---|---|
| **X (% massique)** | 0 | 2 | 2 |
| **Composé x** | / | cétone/aldéhyde | dérivé de sulfonamide |
| **Gloss (60°, UB)** | 87,4 | 89,5 | 89,3 |
| **Stabilité au jaunissement** | Faible jaunissement | Fort jaunissement | Jaunissement |
| **Stabilité à la synérèse** | Stable | Stable | Exsudat |
| **Dureté(s)** | 219 | 274 | 198 |
| **Flexibilité (mm)** | 5,7 | 2,8 | 6 |
| **Adhérence** | 0 | 0 | 0 |

La résine "cétone/aldéhyde" est la résine Variplus SK de la société Degussa.
La résine "dérivé de sulfonamide" est la résine Uniplex 108 (Ethyl toluène sulfonamide) de la société Lanxess.

### Exemple 3 (comparatif) : Essais de différentes résines siliconées

Différentes résines siliconées ont été testées (essais 9 à 11) dans des formulations de vernis à ongles à une concentration massique de 2 % :
- DC 1455 (silicone à fonction amine réactive)
- NC 4602 (silice traitée)
- NC 1469 (polyéther de silicone)
Ces trois composés sont commercialisés par la Société Dow Corning.

**Tableau 6**

| | **Essai 1** | **Essai 9** | **Essai 10** | **Essai 11** |
|---|---|---|---|---|
| **X (% massique)** | 0 | 2 | 2 | 2 |
| **Silicone** | | DC 1455 | NC 4602 | NC 1469 |
| **Gloss (60°, UB)** | 87,4 | - | 77,1 film trouble | 87,3 |
| **Stabilité au jaunissement** | Faible jaunissement | Très fort jaunissement | Faible jaunissement | Jaunissement |
| **Stabilité à la synérèse** | Stable | - | - | - |
| **Dureté (s)** | 219 | - | - | - |
| **Flexibilité (mm)** | 5,7 | - | - | - |
| **Adhérence** | 0 | - | - | - |

Les composés NC 1469 et DC 1455 conduisent à des jaunissements de la composition bien plus importants que la composition de référence (essai 1). Le composé aminé DC 1455 conduit après deux jours seulement à une composition ayant pris une couleur marron foncée.

Quant à la composition renfermant le composé NC 4602, le film obtenu est trouble et est donc loin de présenter une amélioration de la brillance.

Il apparaît donc que l'augmentation de la brillance du vernis obtenu par application d'une composition filmogène contenant une résine polyphényl silsesquioxane sans jaunissement ni synérèse de ladite composition est un effet surprenant au vu des résultats obtenus avec les différents composés à base de silice testés dans cet exemple 3.

## Revendications

1. Composition de vernis pour les ongles, renfermant au moins un agent filmogène, à base de nitrocellulose et/ou d'un dérivé de cellulose, un plastifiant et un solvant, **caractérisée en ce qu'**elle comprend au moins 6% massique d'agent filmogène à base de nitrocellulose et/ou d'un dérivé de cellulose, et une résine polyphénylsilsesquioxane en vue d'améliorer la brillance du vernis après son application sur les ongles, ladite résine polyphénylsilsesquioxane étant une molécule non aminée et présentant une température de transition vitreuse Tg supérieure à 30°C.

2. Composition de vernis selon la revendication 1,
**caractérisée en ce que** la masse molaire de la résine polyphénylsilsesquioxane est comprise entre 500 et 20 000 g/mol, de préférence comprise entre 1 000 et 10 000 g/mol, de préférence encore comprise entre 1 500 et 5 000 g /mol.

3. Composition de vernis selon la revendication 1 ou 2,
**caractérisée en ce qu'**elle comprend un solvant organique, dans lequel est soluble la résine polyphényl silsesquioxane, ledit solvant organique étant de préférence choisi parmi les acétates, tels que les acétates d'alkyle.

4. Composition de vernis selon la revendication 3,
**caractérisée en ce que** le solvant organique, dans lequel est soluble la résine polyphényl silsesquioxane, comprend l'acétate d'éthyle et /ou l'acétate de butyle.

5. Composition de vernis selon l'une quelconque des revendications précédentes,
**caractérisée en ce que** la résine polyphényl silsesquioxane présente une température de transition vitreuse Tg supérieure à 35 °C, de préférence supérieure à 40°C.

6. Composition de vernis selon l'une quelconque des revendications précédentes,
**caractérisée en ce que** la résine poly phényl silsesquioxane est formée de plus de 98 % d'unités phénylsilsesquioxane par rapport au nombre total d'unités silsesquioxane de la résine.

7. Composition de vernis selon l'une quelconque des revendications précédentes,
**caractérisée en ce qu'**elle renferme une proportion massique en résine polyphényl silsesquioxane comprise entre 0,1 % et 10 %, de préférence comprise entre 0,5 % et 5 %, de préférence encore entre 1 % et 5 % de la masse totale de la composition de vernis.

8. Composition de vernis selon l'une quelconque des revendications précédentes,
**caractérisée en ce qu'**elle comprend un premier agent filmogène choisi parmi la nitrocellulose ou un dérivé de la cellulose, et un deuxième agent filmogène choisi parmi les résines polyester et/ou epoxy/tosylamide et/ou (méth)acrylique.

9. Composition de vernis selon l'une quelconque des revendications précédentes,
**caractérisée en ce qu'**elle renferme au moins un solvant choisi parmi les acétates d'alkyle, un premier agent filmogène choisi parmi la nitrocellulose ou un dérivé de la cellulose, un deuxième agent filmogène polyester comprenant le copolymère anhydride trimellitique/acide adipique/néopentyl glycol, et une proportion massique en polyphényl silsesquioxane comprise entre 0,1 % et moins de 10 % de la masse totale de la composition de vernis.

10. Composition de vernis selon l'une quelconque des revendications 8 ou 9,
**caractérisée en ce qu'**elle comprend de 60 à 90 % massique de solvant, de 6 à 30 % massique du premier agent filmogène, de 2 à 20 % massique du deuxième agent filmogène et de 0,1 % à moins de 10 % massique, de préférence de 0,5 à 5 % massique, de polyphényl silsesquioxane, par rapport à la masse totale de la composition de vernis.

11. Composition de vernis selon l'une quelconque des revendications 8 à 10,
**caractérisée en ce qu'**elle comprend de 60 à 80 % massique de solvant, de 8 à 25 % massique du premier agent filmogène, de 5 à 15 % massique du deuxième agent filmogène et de 0,1 % à 9 % massique, de préférence de 0,5 à 5 % massique de polyphényl silsesquioxane, par rapport à la masse totale de la composition de vernis.

12. Composition de vernis selon l'une quelconque des revendications précédentes,
**caractérisée en ce qu'**elle renferme une teneur en benzène inférieure à 2 ppm, de préférence inférieure à 1 ppm.

## Patentansprüche

1. Lackzusammensetzung für die Nägel, die mindestens ein filmbildendes Mittel auf der Basis von Nitrocellulose und/oder einem Cellulosederivat, einen Weichmacher und ein Lösungsmittel einschließt, **dadurch gekennzeichnet, dass** sie mindestens 6 Ma% filmbildendes Mittel auf der Basis von Nitrocellulose und/oder einem Cellulosederivat umfasst, und ein Polyphenylsilsesquioxanharz zur Verbesserung des Glanzes des Lacks nach seinem Auftragen auf die Nägel, wobei das Polyphenylsilsesquioxanharz ein Nicht-Amin-Molekül ist und eine Glasübergangstemperatur Tg von über 30 °C aufweist.

2. Lackzusammensetzung nach Anspruch 1,
**dadurch gekennzeichnet, dass** die molare Masse des Polyphenylsilsesquioxanharzes zwischen 500 und 20.000 g/mol, vorzugsweise zwischen 1.000 und 10.000 g/mol, noch vorzugsweiser zwischen 1.500 und 5.000 g/mol liegt.

3. Lackzusammensetzung nach Anspruch 1 oder 2,
**dadurch gekennzeichnet, dass** sie ein organisches Lösungsmittel umfasst, in welchem das Polyphenylsilsesquioxanharz löslich ist, wobei das organische Lösungsmittel vorzugsweise aus den Acetaten wie den Alkylacetaten ausgewählt ist.

4. Lackzusammensetzung nach Anspruch 3,
**dadurch gekennzeichnet, dass** das organische Lösungsmittel, in dem das Polyphenylsilsesquioxanharz löslich ist, Ethylacetat und/oder Butylacetat umfasst.

5. Lackzusammensetzung nach einem der vorangehenden Ansprüche,
**dadurch gekennzeichnet, dass** das Polyphenylsilsesquioxanharz eine Glasübergangstemperatur Tg von über 35 °C, vorzugsweise von über 40 °C, aufweist.

6. Lackzusammensetzung nach einem der vorangehenden Ansprüche,
**dadurch gekennzeichnet, dass** das Polyphenylsilsesquioxanharz von mehr als 98 % Polyphenylsilsesquioxaneinheiten in Bezug auf die Gesamtanzahl von Silsesquioxaneinheiten des Harzes gebildet ist.

7. Lackzusammensetzung nach einem der vorangehenden Ansprüche,
**dadurch gekennzeichnet, dass** sie einen Massenanteil an Polyphenylsilsesquioxanharz einschließt, der zwischen 0,1 % und 10 %, vorzugsweise zwischen 0,5 % und 5 %, noch vorzugsweiser zwischen 1 % und 5 % der Gesamtmasse der Lackzusammensetzung liegt.

8. Lackzusammensetzung nach einem der vorangehenden Ansprüche,
**dadurch gekennzeichnet, dass** sie ein erstes filmbildendes Mittel umfasst, das aus der Nitrocellulose oder einem Cellulosederivat ausgewählt ist, und ein zweites filmbildendes Mittel, das aus den Polyester- und/oder Epoxy/Tosylamid- und/oder (Meth)acrylharzen ausgewählt ist.

9. Lackzusammensetzung nach einem der vorangehenden Ansprüche,
**dadurch gekennzeichnet, dass** sie mindestens ein Lösungsmittel einschließt, das aus den Alkylacetaten ausgewählt ist, ein erstes filmbildendes Mittel, das aus der Nitrocellulose oder einem Cellulosederivat ausgewählt ist, wobei ein zweites polyester-filmbildendes Mittel das Copolymer Trimellitsäureanhydrid/Adipinsäure/Neopentylglycol umfasst, und einen Massenanteil an Polyphenylsilsesquioxan, der zwischen 0,1 % und weniger als 10 % der Gesamtmasse der Lackzusammensetzung liegt.

10. Lackzusammensetzung nach einem der Ansprüche 8 oder 9,
**dadurch gekennzeichnet, dass** sie 60 bis 90 Ma% Lösungsmittel, 6 bis 30 Ma% des ersten filmbildenden Mittels, 2 bis 20 Ma% des zweiten filmbildenden Mittels und 0,1 bis weniger als 10 Ma%, vorzugsweise 0,5 bis 5 Ma%, Polyphenylsilsesquioxan in Bezug auf die Gesamtmasse der Lackzusammensetzung umfasst.

11. Lackzusammensetzung nach einem der Ansprüche 8 bis 10,
**dadurch gekennzeichnet, dass** sie 60 bis 80 Ma% Lösungsmittel, 8 bis 25 Ma% des ersten filmbildenden Mittels, 5 bis 15 Ma% des zweiten filmbildenden Mittels und 0,1 bis 9 Ma%, vorzugsweise 0,5 bis 5 Ma% Polyphenylsilsesquioxan in Bezug auf die Gesamtmasse der Lackzusammensetzung umfasst.

12. Lackzusammensetzung nach einem der vorangehenden Ansprüche,
**dadurch gekennzeichnet, dass** sie einen Benzolgehalt von unter 2 ppm, vorzugsweise von unter 1 ppm, einschließt.

## Claims

1. Nail varnish composition comprising at least one nitrocellulose and/or cellulose derivative film-forming agent, a plasticizer and a solvent,
**characterized in that** it comprises at least 6 weight % of nitrocellulose and/or cellulose derivative film-forming agent, and a polyphenyl silsesquioxane resin for the purpose of improving the gloss of the varnish after application to nails, said polyphenyl silsesquioxane resin being a non-aminated molecule and having a glass transition temperature Tg higher than 30° C.

2. The varnish composition according to claim 1,
**characterized in that** the molar mass of the polyphenyl silsesquioxane resin is between 500 and 20 000 g/mol, preferably between 1 000 and 10 000 g/mol, further preferably between 1 500 and 5 000 g/mol.

3. The varnish composition according to claim 1 or 2,
**characterized in that** it comprises an organic solvent in which the polyphenyl silsesquioxane resin is soluble, said organic solvent preferably being selected from among acetates such as alkyl acetates.

4. The varnish composition according to claim 3,
**characterized in that** the organic solvent in which the polyphenyl silsesquioxane resin is soluble comprises ethyl acetate and/or butyl acetate.

5. The varnish composition according to any of the preceding claims, **characterized in that** the polyphenyl silsesquioxane resin has a glass transition temperature Tg higher than 35° C, preferably higher than 40° C.

6. The varnish composition according to any of the preceding claims, **characterized in that** the polyphenyl silsesquioxane resin is formed of more than 98 % of phenyl silsesquioxane units relative to the total number of silsesquioxane units of the resin.

7. The varnish composition according to any of the preceding claims, **characterized in that** it contains a weight proportion of polyphenyl silsesquioxane resin of between 0.1 % and 10 %, preferably between 0.5 % and 5 %, more preferably between 1 % and 5 % of the total weight of the varnish composition.

8. The varnish composition according to any of the preceding claims, **characterized in that** it comprises a first film-forming agent selected from among nitrocellulose or a cellulose derivative, and a second film-forming agent selected from among polyester and/or tosylamide/epoxy and/or (meth)acrylic resins.

9. The varnish composition according to any of the preceding claims, **characterized in that** it comprises at least one solvent selected from among alkyl acetates, a first film-forming agent selected from among nitrocellulose or a cellulose derivative, a second polyester film-forming agent comprising the adipic acid/neopentyl glycol/trimellitic anhydride copolymer, and a weight proportion of polyphenyl silsesquioxane of between 0.1 % and less than 10 % of the total weight of the varnish composition.

10. The varnish composition according to any of claims 8 or 9, **characterized in that** it comprises from 60 to 90 weight % of solvent, from 6 to 30 weight % of the first film-forming agent, from 2 to 20 weight % of the second film-forming agent and from 0.1 to less than 10 weight % preferably 0.5 to 5 weight % of polyphenyl silsesquioxane relative to the total weight of the varnish composition.

11. The varnish composition according to any of claims 8 to 10, **characterized in that** it comprises from 60 to 80 weight % of solvent, from 8 to 25 weight % of the first film-forming agent, from 5 to 15 weight % of the second film-forming agent and from 0.1 to 9 weight %, preferably 0.5 to 5 weight % of polyphenyl silsesquioxane relative to the total weight of the varnish composition.

12. The varnish composition according to any of the preceding claims, **characterized in that** it has a benzene content of less than 2 ppm, preferably less than 1 ppm.
